# EUROPEAN PATENT APPLICATION

(11) **EP 4 696 228 A1**
(43) Date of publication of application: **18.02.2026**
(21) Application number: 24194473.5
(22) Date of filing: 14.08.2024
(51) Int. Cl.: A61B 5/024, A61B 5/1455, A61B 5/00

(54) **PHOTOPLETHYSMOGRAPHY SENSOR**

(71) Applicant: Koninklijke Philips N.V., 5656 AG Eindhoven (NL)
(72) Inventor: FRANCK, Christoph Florian, 5656 AG Eindhoven (NL)
(74) Representative: Philips Intellectual Property & Standards

(57) **Abstract**

Proposed concepts aim to provide methods and systems pertaining to a PPG monitoring system comprising a PPG sensor, a processing arrangement, and a control arrangement. In particular, the system is configured to control the emission of optical radiation from an optical source arrangement of the PPG sensor, based on a cardiac value determined from cardiac data that describes at least two cardiac cycles of the subject.

## Description

### FIELD OF THE INVENTION

This invention relates to the field of Photoplethysmography (PPG) sensors and more particularly to PPG monitoring systems.

### BACKGROUND OF THE INVENTION

PPG monitoring systems may be used to obtain vital sign measurements of a subject. PPG systems and devices use optical measurements to detect blood volume changes within a subject's tissue. The detected signals can then be used to calculate the values of various clinical parameters such as heart rate, respiration rate, and blood oxygen saturation (SpO₂). A PPG sensing device typically takes the form of a clip or sleeve sensor that is attached to a subject's finger or another part of the body with good blood supply, such as an earlobe. One or more optical radiation sources are used to emit optical radiation to the subject's tissue, and then an optical detector is used to detect this radiation once it has interacted with the bodily tissue. Pulse oximetry is a specific form of PPG sensing widely used in clinical practice for measuring the oxygen saturation of a subject's arterial blood. PPG monitoring systems and methods are advantageous as they are non-invasive, safe, quick, and convenient.

The accuracy and precision of clinical values calculated or determined from the PPG signal may be highly dependent on the power used by the device since this may impact light intensities, the power draw of operational amplifiers, sampling rates and sampling resolutions. For ease of use, PPG monitoring systems may be battery-powered devices. Power consumption is therefore an important factor both for ensuring a high quality PPG signal and achieving required device longevity and device size. A significant portion of the total power consumption of a pulse oximeter is used for driving the optical radiation sources. It is therefore beneficial to provide power-efficient mechanisms for driving optical radiation sources of PPG monitoring systems.

### SUMMARY OF THE INVENTION

The invention is defined by the claims.

According to examples in accordance with an aspect of the invention, there is provided a PPG monitoring system comprising: a PPG sensor comprising an optical source arrangement; a processing arrangement configured to: obtain cardiac data describing cardiac activity of a subject in a predetermined time period; and determine, based on the obtained cardiac data, a cardiac value of the subject comprising a statistical measure of the cardiac activity of the subject in the predetermined time period; and a control arrangement configured to control the optical source arrangement of the PPG sensor to emit optical radiation, based on the determined cardiac value.

Proposed concepts thus aim to provide schemes, solutions, concepts, designs, methods, and systems pertaining to a PPG monitoring system comprising a PPG sensor, a processing arrangement and a control arrangement. In particular, the system is configured to control the emission of optical radiation from an optical source arrangement of the PPG sensor, based on a cardiac value that describes a statistical measure of the subject's cardiac activity over a predetermined time period.

Typically, pulse oximeters record and process optical data continuously, digitally sampling channels of optical detectors with a constant, uniform sampling rate. This results in a PPG waveform that is easy to process using common signal processing techniques like linear digital filters which presuppose uniformly-sampled signals. However, typical processing steps carried out on PPG signals to determine clinical values such as blood oxygen saturation do not require continuous PPG signals. Therefore, uniform sampling results in energy being spent to require redundant information, reducing the power-efficiency of the device.

PPG sensing devices may have a limit on the average power they expose a subject to in the form of light and heat. Further limits on power consumption may be imposed in battery-powered devices in order to ensure sufficiently long battery lifetimes and sufficiently small device dimensions. However, in general, spending more power in the form of high light intensities, higher power draw of operation amplifiers, higher sampling rates, or higher sampling resolution results in a more accurate measurement of the PPG signal with a higher signal-to-noise ratio (SNR). Therefore, there is a trade-off between achieving accurate clinical measurements and reducing power consumption. It was realised that allocating more of the available power budget to acquire shorter segments of a PPG signal could improve the overall quality of the values derived from the PPG signal, whilst conforming to power consumption limits.

The proposed invention uses a determined cardiac value of the subject to control the emission of optical radiation from a PPG sensor. This may result in a more informed and efficient use of the power budget of a PPG sensor so as to achieve power savings whilst also achieving the required accuracy of the measured PPG signal. In particular, the proposed invention involves the determination of a cardiac value that describes a statistical measure of the cardiac activity of the subject. Hence, the proposed invention uses compiled information about the subject's cardiac activity, describing for example average cardiac parameter values or statistical measures of cardiac activity, to determine the emission characteristics of the sensor. The use of compiled information in this way is advantageous in that it reduces the requirement for low-latency transmission of data. A statistical measure of cardiac activity provides an accurate indication of subject cardiac activity whilst remaining insensitive to large latency values or large variations in latency values associated with the calculation and transmission of the cardiac value and the subsequent control of the PPG sensor. Hence, the proposed invention results in power-efficient PPG monitoring that permits accurate determination of clinical values without placing constraints on the latency associated with the various aspects of the system.

Ultimately, an improved system for PPG monitoring of a subject may therefore be supported by the proposed concept(s).

In some embodiments, the predetermined time period corresponds to at least two cardiac cycles of the subject. This may permit the cardiac value to more reliably represent the cardiac activity of the subject.

In some embodiments, the cardiac data may comprise a plurality of cardiac data values, each cardiac data value describing the cardiac activity of the subject in a respective time period. Determining the cardiac value of the patient may then comprise: determining based on the cardiac data, a distribution of the plurality of cardiac data values; and determining the cardiac value of the subject based on calculating the value of a statistical parameter of the determined distribution of the plurality of cardiac data values. Therefore, cardiac data of various forms describing the cardiac activity of the subject over time may be used to derive a statistical parameter of the subject's cardiac cycle. This can provide useful insights for controlling the emission of the optical source arrangement of the PPG sensor to be more power-efficient.

In some embodiments, controlling the optical source arrangement may comprise determining, based on the cardiac value, a value of an emission parameter of the optical source arrangement; and controlling the optical source arrangement to emit optical radiation, based on the determined value of the emission parameter, such that the optical source arrangement emits optical radiation for at least a predetermined portion of the cardiac cycle of the subject. Thus, the cardiac value may be used to predict values of emission parameters of the optical source arrangement that are likely to allow for a predetermined portion of the cardiac cycle to be captured by the PPG sensor. This may ensure an improved trade-off is achieved between reducing power consumption and ensuring sufficient accuracy of clinical values calculated from the measured PPG signal.

In some embodiments, controlling the optical source arrangement may comprise adjusting an emission parameter of the optical source arrangement, wherein the optical source arrangement comprises at least one of: a duration of emission of optical radiation; a pattern of emission of optical radiation; an intensity of emission of optical radiation; and a duty cycle of optical radiation. Informed control of any of these parameters based on information about the subject's cardiac cycle may reduce the power-consumption of the PPG sensor.

In some embodiments, the cardiac value may comprise at least one of: an average heart rate; a maximum heart rate; a minimum heart rate; a variability in the heart rate; a measure of QRS complex duration; a measure of QT interval duration; and a measure of PR interval duration. These cardiac parameters may be easily measured at low energy cost and provide sufficient information about the subject's cardiac cycle to allow for power-efficient control of the optical source arrangement of the PPG sensor to emit optical radiation.

In some embodiments, the cardiac data may comprise at least one of: an ECG signal; and blood pressure data of the subject. ECG measurements and blood pressure measurements may be commonly taken alongside pulse oximetry measurements as part of multi-parameter patient monitors and therefore cardiac data in these forms may be easy to obtain and analyse.

In some embodiments, the optical source arrangement may comprise a plurality of optical sources, and controlling the optical source arrangement of the PPG sensor to emit optical radiation may comprise controlling each of the plurality of optical sources of the optical source arrangement independently. PPG sensors may be equipped with multiple optical sources at different positions and/or with different emission properties. Controlling the emission of each source independently may allow for greater control over the balance between power-efficiency and the accuracy of the measured PPG signal, as for example different sources may be configured to emit at different times.

In some embodiments, each of the plurality of optical sources may be configured to emit optical radiation within a different wavelength range. Multi-wavelength measurements may be often used for pulse oximetry as different wavelengths of light interact with oxygenated and deoxygenated blood in different ways. The duration of a PPG signal required for further processing may vary depending on the wavelength of radiation used. Therefore, controlling each source associated with a different wavelength band differently allows for improved accuracy of clinical values determined based on the measured PPG signals.

In some embodiments, the optical source arrangement may comprise at least one optical source, and controlling the optical source arrangement of the PPG sensor to emit optical radiation may comprise deactivating an optical source of the optical source arrangement for a predetermined time period. Thus, in some proposed systems, individual optical sources may be deactivated. This provides a simple and efficient way of controlling the intensity of the emission of the PPG sensor within different time intervals and providing energy savings compared to continuous uniform sampling.

In some embodiments, deactivating an optical source may comprise at least one of: powering down the optical source; and powering down analogue signal acquisition circuitry and an analogue-to-digital converter of the PPG sensor associated with the optical source. These are easy and efficient mechanisms for switching off the emission of an optical radiation source.

According to an aspect of the present invention, there is provided a subject monitoring system. The subject monitoring system comprises, a cardiac monitoring device configured to acquire cardiac data describing cardiac activity of a subject; and the PPG monitoring system of any of the above described embodiments.

According to another aspect of the present invention, there is provided a method for operating a PPG monitoring system, wherein the PPG monitoring system comprises a PPG sensor comprising an optical source arrangement. The method comprises: obtaining cardiac data describing cardiac activity of a subject in a predetermined time period; determining, based on the obtained cardiac data, a cardiac value of the subject comprising a statistical measure of the cardiac activity of the subject in the predetermined time period; and controlling the optical source arrangement of the PPG sensor to emit optical radiation based on the determined cardiac value.

Embodiments may be employed in combination with conventional/existing PPG sensing methods and systems. In this way, embodiments may integrate into legacy systems to improve and/or extend their functionality and capabilities. An improved PPG monitoring system with improved power-efficiency may therefore be provided by the proposed embodiments.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of a PPG monitoring system according to a proposed embodiment;
Fig. 2 is a simplified illustration of a PPG sensor;
Figs. 3A and 3B depict PPG gating signals that may be employed by a proposed PPG monitoring system;
Fig. 4 is a simplified block diagram of a subject monitoring system according to an aspect of the present invention; and
Fig. 5 is a simplified flow diagram of a method for operating a PPG monitoring system according to another aspect of the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figs. are merely schematic and are not drawn to scale. It should also be understood that the same reference numerals are used throughout the Figs. to indicate the same or similar parts.

Implementations in accordance with the present disclosure relate to various techniques, methods, schemes, and/or solutions pertaining to PPG monitoring systems. According to proposed concepts, several possible solutions may be implemented separately or jointly. That is, although these possible solutions may be described below separately, two or more of these possible solutions may be implemented in one combination or another.

Embodiments of the invention aim to provide a PPG monitoring system in which the emission of optical radiation by an optical source arrangement of a PPG sensor is controlled based on a cardiac value of the subject. This may be used beneficially to reduce the power consumption of the PPG sensor (allowing for a longer run-time in the case of battery-operated devices), whilst maintaining the accuracy and availability of common clinical values derived from the PPG signal.

PPG monitoring involves illuminating a part of a subject's body that has good blood supply with optical radiation and detecting the radiation once it has interacted with the subject's blood. This detected radiation forms the photoplethysmography (PPG) signal. PPG-based pulse oximetry relies on the concept that haemoglobin, the oxygen-carrying molecule of the blood, has different light absorption properties in its oxygenated and deoxygenated state.
These differences, and the resulting difference in the detected optical radiation can ultimately be used to calculate the ratio of oxygenated to total haemoglobin in the arterial, pulsatile volume of blood in a given bodily tissue of the subject.

The present invention is based on the insight that not all parts of PPG waveforms recorded by PPG sensors are equally relevant for deriving values such as the blood oxygen saturation or respiration rate. It is therefore not necessary to continuously measure a PPG signal in order to obtain accurate measurements of these values as the PPG signal is required to capture only a portion of the full cardiac cycle.

Multi-parameter monitors typically contain both one or more PPG sensors and additional vital signs sensors that provide information about the cardiac cycle. This may include for example electrocardiography sensors (ECG sensors) or blood pressure sensors (for example, invasive arterial blood pressure sensors). In the proposed invention, cardiac data from such sensors is used to obtain compiled information about the subject's cardiac cycle that indicates a statistical measure of the cardiac activity of the subject. Illumination parameters of a PPG sensor are then modified and controlled based on this information about the subject's cardiac cycle. This may result in improved PPG sensing with greater power-efficiency.

Since the cardiac value describes a statistical measure of the cardiac activity of the subject, the proposed PPG sensing system is not subject to latency constraints. It is not necessary for the cardiac data to be available substantially instantaneously at the control arrangement for control of the PPG sensor since the cardiac value describes the cardiac activity over a predetermined length of time. Therefore, the proposed system can therefore tolerate latency of cardiac value updates that fluctuates in a range of several hundred milliseconds, allowing for the proposed system to be utilised in a wide range of patient monitoring systems and devices.

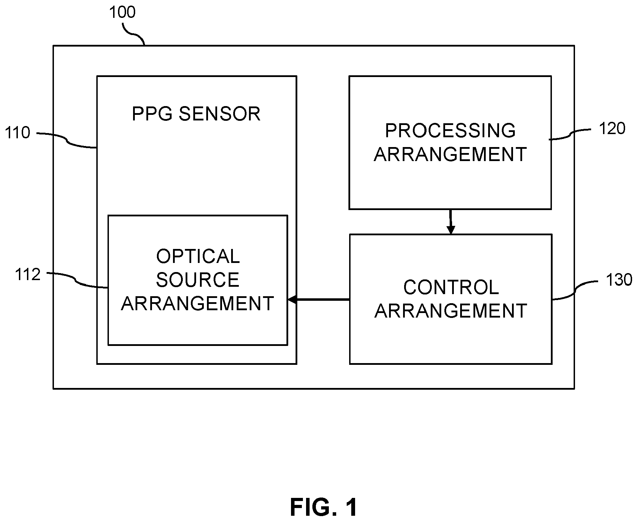

Referring now to Fig. 1 there is depicted a PPG monitoring system 100 according to a proposed embodiment.

The PPG monitoring system 100 comprises a PPG sensor 110 which is configured to take optical measurements of a subject's bodily tissue. The PPG sensor comprises an optical source arrangement 112.

The PPG monitoring system 100 further comprises a processing arrangement 120 configured to obtain cardiac data describing cardiac activity of a subject in a predetermined time period. In this exemplary embodiment, the cardiac data comprises an ECG signal corresponding to several cardiac cycles.

The processing arrangement 120 is further configured to determine, based on the obtained cardiac data, a cardiac value of the patient, the cardiac value comprising a statistical measure of the cardiac activity of the subject in the predetermined time period. In this exemplary embodiment, the cardiac value comprises the subject's average heart rate over the several cardiac cycles described by the ECG signal.

Once the average heart rate of the subject has been determined, a control arrangement 130 of the PPG monitoring system 100 is configured to control the optical source arrangement of the PPG sensor to emit optical radiation, based on the determined cardiac value.

As an example, the optical source arrangement 112 of the PPG sensor 110 is configured to emit pulses of light. The duration of each pulse of emitted optical radiation and the time between separate pulses is controlled by the control arrangement 130 depending on the determined average heart rate of the subject. Higher heart rates result in the optical source arrangement being controlled to emit shorter pulses of light and a reduction in the periodicity of the pulses. The duration of the pulse and the time between pulses may be calculated such that it is statistically likely, based on the determined heart rate of the subject, that the pulse captures a sufficient fraction of the cardiac cycle of the subject for an accurate determination of the subject's blood oxygen saturation. In other words, controlling the optical source arrangement may comprise determining, based on the cardiac value, a value of an emission parameter of the optical source arrangement; and controlling the optical source arrangement to emit optical radiation, based on the determined value of the emission parameter, such that the optical source arrangement emits optical radiation for at least a predetermined portion of a cardiac cycle of the subject. Therefore, implementation of the proposed invention advantageously allows for non-continuous PPG signal sampling such that only a portion of the cardiac cycle is measured, whilst ensuring sufficient PPG data is gathered to achieve the required accuracy of clinical values calculated based on this data.

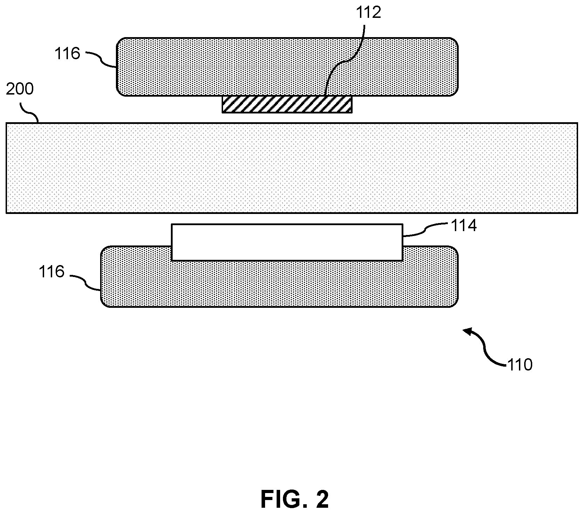

The PPG sensor 110 is depicted in further detail in Fig. 2. In this example, the PPG sensor is a transmission-based sensor comprising the optical source arrangement 112, an optical detector arrangement 114, and a support structure 116. The support structure 116 is configured such that, in use, the optical source arrangement 112 and the optical detector arrangement 114 are positioned on opposite sides of a bodily tissue of the subject 200.

The optical source arrangement 112 is configured to emit optical radiation to the bodily tissue 200. At least a portion of the optical radiation emitted by the optical source arrangement 112 is detected at the optical detector arrangement 114, once it has interacted with the bodily tissue 200. The intensity of the detected optical radiation carries information about properties of the blood within the bodily tissue 200 (since this will affect the optical properties of the bodily tissue) and therefore may be used to derive vital sign measurements.

The PPG sensor 110 shown in Fig. 2 is just one example of a possible PPG sensor of the PPG monitoring system 100. Many other configurations of the PPG sensor 110 are possible. For example, in some instances a reflection-based PPG sensor may be used instead of a transmission-based sensor. In this case the optical source arrangement and the optical detector arrangement are configured to be on the same side of the subject's bodily tissue when in use, such that the optical detector arrangement detects a portion of the light emitted by the optical source arrangement that is internally reflected by the bodily tissue.

Some embodiments may employ optical source arrangements and optical detector arrangements with a plurality of optical sources and a plurality of optical detectors respectively. In particular, the optical source arrangement may comprise a plurality of optical sources wherein each of the plurality of optical sources is configured to emit optical radiation within a different wavelength range. Multi-wavelength PPG sensing can provide further, more detailed insights into the pulsatile properties of blood vessels within the bodily tissue. In this instance the PPG monitoring system 100 may be configured to control each of the plurality of optical sources of the optical source arrangement independently.

Other aspects of the PPG monitoring system 100 described above may also be implemented differently in alternative embodiments. For example, the cardiac value of the subject is not limited to the subject's average heart rate. In other embodiments, the cardiac value may comprise at least one of: a maximum heart rate; a minimum heart rate; a variability in the heart rate; a measure of QRS complex duration; a measure of QT interval duration; and a measure of PR interval duration. In some embodiments, the cardiac value may comprise a statistical measure of the cardiac activity of the subject over a predetermined time period that is calculated directly from the cardiac data. For example, the cardiac data may comprise a plurality of cardiac data values, each cardiac data value describing the cardiac activity of the subject in a respective time period, and determining the cardiac value of the subject may comprise: determining, based on the cardiac data, a distribution of the plurality of cardiac data values; and determining the cardiac value of the subject by calculating the value of a statistical parameter of the determined distribution of the plurality of cardiac data values. Thus, any suitable cardiac value or set of cardiac values that describe compiled information about the subject's cardiac cycle may be used in the proposed systems and methods.

Controlling the optical source arrangement of the PPG sensor to emit optical radiation may take many different forms. As an alternative, or in addition to, the pulse modulation scheme described above, in some embodiments controlling the optical source arrangement may comprise deactivating an optical source of the optical source arrangement for a predetermined time period. The deactivation may involve simply powering down the optical source, or alternatively powering down analogue signal acquisition circuitry and an analogue-to digital converter of the PPG sensor that are associated with the optical source. Deactivation of one or a selection of optical sources of the optical source arrangement may be a simple way in which the emission of the PPG sensor can be controlled at a given time to reduce the power consumption of the system. Controlling this deactivation (e.g., the length of time for which the optical source is deactivated and/or the period of time between successive deactivations) based on a determined cardiac value, may permit this power-reduction to be achieved whilst ensuring it is still possible to achieve the required accuracy of clinical values determined based on the measured PPG signal.

In some embodiments, controlling the optical source arrangement comprises adjusting an emission parameter of the optical source arrangement, wherein the emission parameter comprises at least one of: a duration of emission of optical radiation; a pattern of emission of optical radiation; an intensity of emission of optical radiation; and a duty cycle of optical radiation emission. PPG monitoring systems may use various emission patterns for measuring the PPG signal. Varying the properties of these emission patterns may be used in embodiments of the present invention to improve the power efficiency of a PPG monitoring system. For example, adjusting the emission parameter may comprise at least one of: reducing a total time period for which optical radiation is emitted; reducing the average intensity of emitted radiation; and reducing the maximum intensity of emitted optical radiation.

The PPG monitoring system 100 is described in terms of a single evaluation of the cardiac value and subsequent control of the PPG sensor. In some embodiments, this principle is extended such that cardiac data is obtained at regular or semi-regular intervals. For example, cardiac data describing the last n cardiac cycles of the subject may be obtained at regular time intervals. The cardiac value is then reevaluated based on the most recently available cardiac data. In another embodiment, the cardiac data is compiled and analysed to determine the cardiac value every second or every two seconds. Since the cardiac value described compiled parameters of the subject's cardiac activity, the proposed invention does not require exact synchronisation between the determination of the cardiac value and the control of the optical source arrangement.

Once PPG data has been obtained using any of the proposed PPG monitoring systems of the present invention, this data may be used to determine clinical values such as the blood oxygen concentration. In some embodiments, controlling the emission of optical radiation involves the acquisition of discrete segments of PPG signals as the optical source arrangement is controlled to emit optical radiation for only a portion of the full cardiac cycle. Some analysis methods for pulse oximetry do not require continuous PPG waveforms, however others (such as frequency-domain or simple time-domain methods) do require continuous PPG waveforms. For these methods, a continuous PPG waveform can be reconstructed from the measured discrete PPG segments using predetermined models and templates.

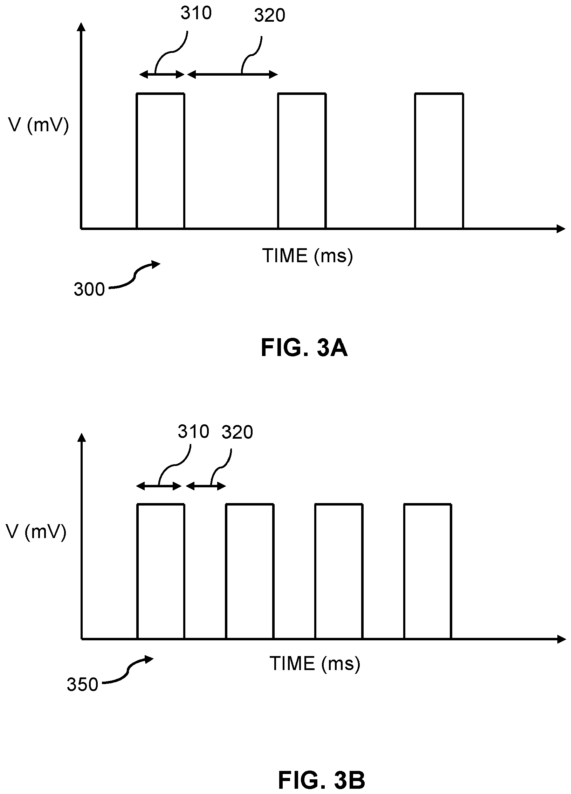

Referring now to Fig. 3A there is depicted an example of a PPG gating signal 300 that may be employed in a proposed PPG monitoring system.

The graph of Fig. 3A depicts a PPG gating signal used to control the optical source arrangement of a PPG sensor to emit optical radiation. The signal comprises a pulse function that controls the optical source arrangement to emit corresponding pulses of optical radiation. The duration 310 of the pulses of the PPG gating signal and the time interval between successive pulses 320 may be controlled based on a determined cardiac value of a monitored subject.

In this example, the average heart rate of the subject in a particular time period is determined to be 60 bpm. In response to this determination, the pulse duration 310 is controlled to have a value of 100 ms, and the interval between successive pulses 320 is controlled to have a value 200 ms.

Fig. 3B depicts a PPG gating signal 350 used to control the same PPG sensor at a different point in time. Over a different predetermined time period, the average heart rate of the subject is determined to be 120 bpm. In response to the increased measured heart rate, the PPG sensor is controlled with an adjusted PPG gating signal 350 for which the interval between successive pulses 320 has been reduced to 100 ms. For a higher heart rate, an increased pulse rate for PPG sensing may be required to ensure sufficient portions of the cardiac cycle of the patient are captured in the measured PPG signal.

Figs. 3A and 3B show just one simple example of how the emission of optical radiation may be controlled based on a determined cardiac value. Many other methods for controlling the PPG sensor are envisioned.

For example, heart rate variability may be used to add a margin to a duration of illumination of the optical source arrangement to ensure that at least one full cardiac cycle is captured during illumination even in the case of significant variation in the duration of the cardiac cycle from beat to beat. This may be used in the case of patients displaying arrythmia for which use of an average heart rate value to control the emission of optical radiation by the PPG sensor may result in certain cardiac cycles not being captured sufficiently.

A minimum heart rate value of the subject may be derived from an average heart rate and a variation in the heart rate, or determined directly, and may be used in a similar manner to the heart rate variability to ensure that a sufficient portion of the cardiac cycle is captured during emission from the optical source arrangement of the PPG sensor to permit the accurate determination of clinical values from the PPG signal.

In some embodiments, a machine learning model may be used to determine an emission parameter for the optical source arrangement of the PPG sensor based on a determined cardiac value. The machine learning model may be trained on a large dataset of training data to take a cardiac value of the subject as an input and output an emission parameter value for the PPG sensor that is likely to result in a sufficient portion of the subject's cardiac cycle being captured in the measured PPG signal for the accurate determination of clinical values.

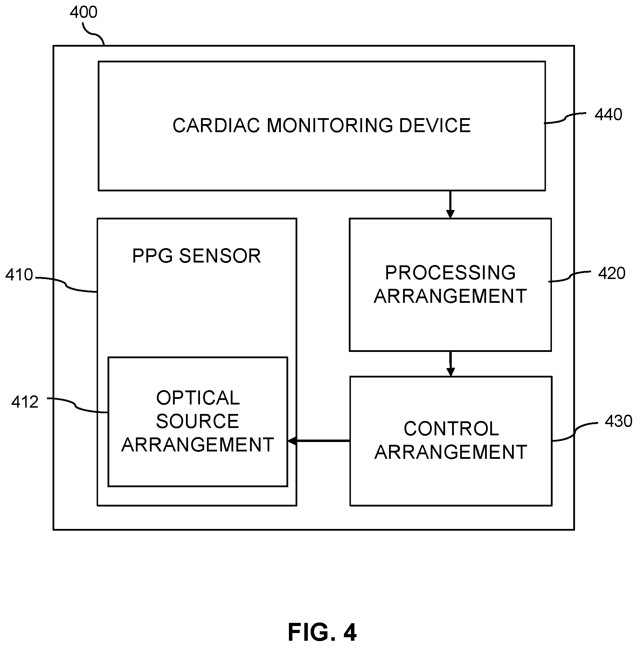

Referring now to Fig. 4 there is depicted a subject monitoring system 400 according to an aspect of the present invention. The subject monitoring system comprises a PPG sensor 410, a processing arrangement 420, and a control arrangement 430. These components are similar to the PPG sensor 110, processing arrangement 120, and control arrangement 130 of the PPG monitoring system 100 and therefore a detailed discussion of these components is not repeated.

The subject monitoring system further comprises a cardiac monitoring device 440. This may be any sensor or combination of sensors that provide cardiac monitoring of a subject. For example, the cardiac monitoring device may comprise at least one of: an ECG sensor; and a blood pressure sensor. The cardiac monitoring device 440 is configured to obtain cardiac data describing cardiac activity of a subject in a predetermined time period.

Once the cardiac data has been obtained it is passed to the processing arrangement 420 and then used to control the optical source arrangement 412 of the PPG sensor 410 via the control arrangement 430 as described above. Fig. 4 thus shows how the concepts of the proposed invention may be integrated into a multi-parameter patient monitoring device.

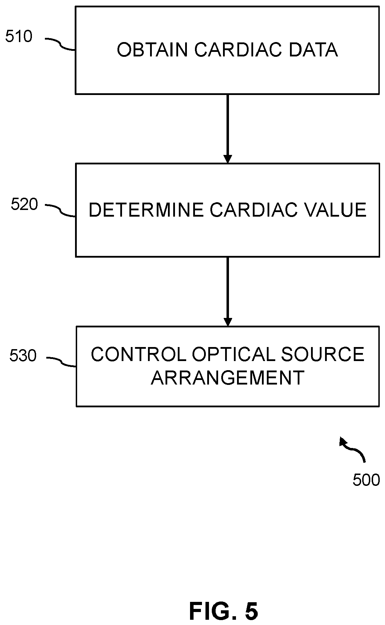

Referring now to Fig. 5, there is depicted a method 500 for operating a PPG monitoring system according to an aspect of the present invention. The PPG monitoring system comprises a PPG sensor comprising an optical source arrangement configured, in use, to emit optical radiation to a bodily tissue of a subject.

The method commences with a step 510 comprising obtaining cardiac data describing cardiac activity of the subject in a predetermined time period. The method then proceeds to a step 520 comprising determining, based on the obtained cardiac data, a cardiac value of the subject comprising a statistical measure of the cardiac activity of the subject in the predetermined time period.

Once the cardiac value has been determined, the method proceeds to step 430 comprising controlling the optical source arrangement of the PPG sensor to emit optical radiation based on the determined cardiac value.

As discussed above, embodiments make use of a control arrangement and a processing arrangement. These components may be implemented in numerous ways, with software and/or hardware to perform the various functions required. A processor is one example of a control component that employs one or more microprocessors that may be programmed using software (e.g., microcode) to perform the required functions. A control arrangement may however be implemented with or without employing a processor, and also may be implemented as a combination or dedicated hardware to perform some functions and a processor (e.g., one or more programmed microprocessors and associated circuitry) to perform other functions.

Examples of control arrangement components that may be employed in various embodiments of the present disclosure include, but are not limited to, conventional microprocessors, application specific integrated circuits (ASICs), and field-programmable gate arrays (FPGAs).

The processing arrangement may be implemented as a combination of dedicated hardware to perform some functions and one or more programmed microprocessors and associated circuitry to perform other functions. Examples of circuitry that may be employed in various embodiments of the present disclosure include, but are not limited to, conventional microprocessors, application specific integrated circuits (ASICs), and field-programmable gate arrays (FPGAs).

In various implementations, the processing arrangement and the control arrangement may be associated with one or more storage media such as volatile and non-volatile computer memory such as RAM, PROM, EPROM, and EEPROM. The storage media may be encoded with one or more programs that, when executed on one or more processing arrangements and/or control arrangements, perform the required functions. Various storage media may be fixed within a processing arrangement or control arrangement or may be transportable, such that the one or more programs stored thereon can be loaded into a processor or controller.

The systems of Figs. 1 and 4, and the method of Fig. 5 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flow charts - may be performed by the same or different computing devices.

To the extent that an embodiment is implemented partly or wholly in hardware the blocks shown in the block diagrams of Figs. 1 and 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium such as an optical storage medium or a solid-state medium supplied together with or as part of other hardware, but may also be distributed in other forms, such as via the Internet or other wired or wireless telecommunication systems.

The flow chart and block diagrams in the Figs. illustrate the architecture, functionality, and operation of possible implementations of systems, methods, and computer program products according to various embodiments of the present invention. In this regard each block in the flow charts or block diagrams may represent a module segment or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combination of blocks in the block diagrams and/or flow chart illustrations, can be implemented by special purpose hardware-based systems that perform the specified functions.

Variations to the disclosed embodiments can be understood and effected by those skilled in the art in practicing the claimed invention, from a study of the drawings the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If the term "adapted to" is used in the claims or description it is noted the term "adapted to" is intended to be equivalent to the term "configured to". Any reference signs in the claims should not be construed as limiting the scope.

## Claims

1. A PPG monitoring system (100) comprising:
a PPG sensor (110) comprising an optical source arrangement (112);
a processing arrangement (120) configured to:
obtain cardiac data describing cardiac activity of a subject in a predetermined time period; and
determine, based on the obtained cardiac data, a cardiac value of the subject comprising a statistical measure of the cardiac activity of the subject in the predetermined time period; and
a control arrangement (130) configured to control the optical source arrangement of the PPG sensor to emit optical radiation, based on the determined cardiac value.

2. The PPG monitoring system of claim 1, wherein the predetermined time period corresponds to at least two cardiac cycles of the subject.

3. The PPG monitoring system of any preceding claim, wherein the cardiac data comprises a plurality of cardiac data values, each cardiac data value describing the cardiac activity of the subject in a respective time period, and
wherein determining the cardiac value of the subject comprises:
determining, based on the cardiac data, a distribution of the plurality of cardiac data values; and
determining the cardiac value of the subject by calculating the value of a statistical parameter of the determined distribution of the plurality of cardiac data values.

4. The PPG monitoring system of any preceding claim, wherein controlling the optical source arrangement comprises:
determining, based on the cardiac value, a value of an emission parameter of the optical source arrangement; and
controlling the optical source arrangement to emit optical radiation, based on the determined value of the emission parameter, such that the optical source arrangement emits optical radiation for at least a predetermined portion of a cardiac cycle of the subject.

5. The PPG monitoring system of any preceding claim, wherein controlling the optical source arrangement comprises adjusting an emission parameter of the optical source arrangement, wherein the emission parameter comprises at least one of:
a duration of emission of optical radiation;
a pattern of emission of optical radiation;
an intensity of emission of optical radiation; and
a duty cycle of optical radiation emission.

6. The PPG monitoring system of any preceding claim, wherein the cardiac value comprises at least one of:
an average heart rate;
a maximum heart rate;
a minimum heart rate;
a variability in the heart rate;
a measure of QRS complex duration;
a measure of QT interval duration; and
a measure of PR interval duration.

7. The PPG monitoring system of any preceding claim, wherein the cardiac data comprises at least one of:
an ECG signal; and
blood pressure data of the subject.

8. The PPG monitoring system of any preceding claim, wherein the optical source arrangement comprises a plurality of optical sources, and
wherein controlling the optical source arrangement of the PPG sensor to emit optical radiation comprises controlling each of the plurality of optical sources of the optical source arrangement independently.

9. The PPG monitoring system of claim 8, wherein each of the plurality of optical sources is configured to emit optical radiation within a different wavelength range.

10. The PPG monitoring system of any preceding claim, wherein the optical source arrangement comprises at least one optical source, and
wherein controlling the optical source arrangement of the PPG sensor to emit optical radiation comprises deactivating an optical source of the optical source arrangement for a predetermined time period.

11. The PPG monitoring system of claim 10, wherein deactivating an optical source comprises at least one of:
powering down the optical source; and
powering down analogue signal acquisition circuitry and an analogue-to-digital converter of the PPG sensor associated with the optical source.

12. A subject monitoring system (400), comprising:
a cardiac monitoring device (440) configured to acquire cardiac data describing cardiac activity of a subject; and
the PPG monitoring system of any of claims 1-10.

13. A method (500) for operating a PPG monitoring system, wherein the PPG monitoring system comprises a PPG sensor comprising an optical source arrangement, the method comprising:
obtaining (510) cardiac data describing cardiac activity of a subject in a predetermined time period;
determining (520), based on the obtained cardiac data, a cardiac value of the subject comprising a statistical measure of the cardiac activity of the subject in the predetermined time period; and
controlling (530) the optical source arrangement of the PPG sensor to emit optical radiation, based on the determined cardiac value.

14. The method of claim 13, wherein the predetermined time period corresponds to at least two cardiac cycles of the subject.

15. The method of any of claims 13 and 14, wherein controlling the optical source arrangement comprises adjusting an emission parameter of the optical source arrangement, wherein the emission parameter comprises at least one of:
a duration of emission of optical radiation;
a pattern of emission of optical radiation
an intensity of emission of optical radiation; and
a duty cycle of optical radiation emission.
